# EUROPEAN PATENT SPECIFICATION

(11) **EP 3 237 635 B1**
(45) Date of publication and mention of the grant of the patent: **23.10.2019**
(21) Application number: 15816856.7
(22) Date of filing: 22.12.2015
(51) Int. Cl.: C12Q 1/6853, C12Q 1/6869

(54) **METHOD FOR GENERATING SEQUENCE READY FRAGMENTS BY THE USE OF "BUBBLE PRIMERS"**
VERFAHREN ZUR HERSTELLUNG VON SEQUENZIERBEREITEN FRAGMENTEN MIT HILFE VON "BUBBLE PRIMER"
PROCÉDÉ DE PREPARATION DES FRAGMENTS DE SÉQUENCAGE PAR "BUBBLE PRIMERS"

(30) Priority: 22.12.2014 GB 201422982
(43) Date of publication of application: 01.11.2017
(73) Proprietor: DNAE Group Holdings Ltd, London W12 7SB (GB)
(72) Inventor: MCKEOWN, Brian James, London W12 7SB (GB); MCELGUNN, Cathal Joseph, London W12 7SB (GB)
(74) Representative: Stratagem IPM Limited
(86) International application number: PCT/GB2015/054125
(87) International publication number: WO 2016/102956

(56) References cited:
- EP-A1- 2 770 090
- WO-A1-2012/103154
- WO-A2-2015/081229
- US-A1- 2007 077 570
- US-A1- 2012 171 673
- US-A1- 2013 184 165
- CAIFU CHEN ET AL: "Real-time quantification of Micro-RNAs by stem-loop RT-PCR", NUCLEIC ACIDS RESEARCH, INFORMATION RETRIEVAL LTD, GB, vol. 33, no. 20, 1 January 2005 (2005-01-01), page E179, XP008132134, ISSN: 0305-1048, DOI: 10.1093/NAR/GNI178

## Description

### FIELD OF THE INVENTION

The present invention relates to a method for generation of polynucleotide fragments from a starting template that are amenable to DNA sequencing analysis. The fragments are of use in next generation sequencing methods. Aspects of the disclosure relate to nucleic acid primers for use in such a method.

### BACKGROUND TO THE INVENTION

Since the completion of the draft human genome sequence, the biochemistry and instrumentation of DNA sequencing analysis has advanced to the point that for the same financial outlay lavished on the original genome, it would now (2014) be possible to generate the genome sequences of every man, woman and child in metropolitan Chicago (population 2.7 million) at a rate of one complete genome per 29 hours per instrument, with 30x coverage of each and every region of the genome. This phenomenal increase in capacity is due to the ability to treat all of the fragments of DNA being sequenced in a generic manner, with each portion of the genome being exposed to the same biochemistry at the same time. 'Massively parallel' DNA sequencing is enabled by the random fragmentation of the genomic DNA and then the enzymatic attachment of artificial sequence 'adapters' to each end of the pieces of fragmented DNA.

Generating a sequencing 'library' from genomic DNA is time consuming, and generates fragments in which about half of the templates are actually not amenable for analysis, due to the random nature of the attachment of two different 'flavours' of adapter: many products will have identical 'type A' or 'type B' adapters attached at the ends of the fragment, whereas what is required is fortuitous asymmetric attachment, with one flavour of adapter (type A) on one end and the other flavour (type B) of the adapter on the other end. These asymmetric products are capable of being clonally amplified and are ideal for generating valuable sequence information from genomic template as soon as the sequencing reaction commences.

Current art in sequencing library preparation for NGS includes the steps of:
- Random fragmentation of the template DNA
- Size selection of those fragments of a desired length
- Enzymatic 'end repair' of ends of fragments to allow blunt-end ligation of Type A and Type B adapters
- Ligation of adapters, generating a proportion of 'A/A' and 'B/B' redundant products, and a population of 'A/B' desirable product
- Clonal amplification of adapter-modified library fragments

As the cost of genome re-sequencing plummets, and the rapidity of sequencing increases, the application of NGS is increasingly turning towards the clinic. However, there will be few circumstances in which it is relevant to read all 3.2 billion bases of the genome; it is likely that a much more targeted approach will be of utility, with therapy being directed by the investigation of a limited number of genetic locations associated with (or perhaps confirming) a specific condition. If it is not necessary to read all of the bases of the genome, then it follows that it may not be optimal to apply technologies and methodologies that have been optimised to achieve just that. EP2770090 A1 describes the use of adaptors for introducing non-target complementary sequences. US200/0077570 A1 and Chen C. et al., Nucleic Acids Res.33, e179, 2005 describe stem-loop primers for modifying 3' ends of micro RNA. US2012/0171673 A1 describes loop primers for introducing a tag sequence into a nucleic acid sample. WO2015/081229 A2 describes a stem loop primer for introducing a probe hybridization site.

The targeted sequencing of specific regions most efficiently requires the isolation of those sequences from the bulk template, which can be extremely diverse and complex. Effectively, this can be achieved by amplifying the target regions to a level that they outnumber the other non-amplified regions. Such amplified products would be amenable to that attachment of NGS terminal adapters (as above), but these would again be a mixed population in which a substantial proportion of the sequences would be 'A/A' and 'B/B' forms, which are inappropriate to support the clonal amplification for NGS sequencing. Critically, even those constructs of type A/B would have a large region of 'primer remnant' present inserted between the adapter sequences and the region of genomic DNA targeted for sequencing. If the adapter sequences provide a binding site for sequencing primers, then these primer remnants will be the first data generated: unnecessary and uninformative. Substantial regions of known sequence must be processed by the NGS method before the unknown sequence of interest is reached. Not only does this use unnecessary resources, but typically sequencing methods are most accurate nearer the start of the read, such that the later unknown sequence is being read with a lower fidelity. It would be desirable to be able to produce sequence fragments with a shorter known region to reduce the amount of unnecessary sequencing which is carried out, and to improve the fidelity of the unknown sequence of interest that is generated.

A further disadvantage of the ligation-based adaptor strategy is that it is necessary to obtain asymmetric integration of the adaptors (that is, a different adaptor on each end of the sequence fragment). However, ligation reactions are typically non-directed, such that only a portion of the fragments will include the necessary asymmetric adaptors; the others will include identical adaptors on either end. It would be desirable to provide a method which allows for inherently asymmetric integration of adaptors.

### SUMMARY OF THE INVENTION

According to a first aspect of the present invention, there is provided a method for generating polynucleotide fragments from a starting template polynucleotide, the method comprising:
a) amplifying a region of interest from the starting template using a first primer pair to form an amplicon incorporating the region of interest,
b) amplifying the region of interest from the first amplicon generated in step a) using a nucleic acid amplification reaction with a second primer, to form an amplicon incorporating the second primer,
wherein the second primer comprises a nucleic acid sequence having a first portion which is complementary to a first portion of the starting template, a second portion which is not complementary to the starting template, and a third portion which is complementary to a second portion of the starting template;
wherein the first and second portions of the starting template are adjacent or in close proximity to one another;
wherein the first, second, and third portions of the second primer are arranged in that order from 5' to 3', such that on hybridisation to the starting template the second portion of the primer remains unhybridised and forms a loop between the first and third portions;
thereby generating an amplified product comprising a region of interest flanked by sequences of the second primer.

The amplified products thereby generated include portions of the second primer, and the method may therefore be used to generate amplicons incorporating known sequences which can be used for sequencing reactions (that is, the products are "sequencing ready").

Preferably the amplification reaction of step b) is carried out with a second primer pair, each of which is of the form of the second primer. In this way, the amplified product includes primer sequences at each end.

The second portion of the second primer comprises a generic sequence; for example, a sequence that is at least substantially identical to sequencing primer sequences. This generic sequence may be common to many or all possible second primers, thereby allowing the amplicon to be used in the sequencing reaction.

The generic sequence may further be adjacent a sequence comprising each of the four nucleotide bases A, C, G, and T. This may be a simple tetrad of four nucleotides (eg, ATCG), or may include two, three, or more of each base (eg, AACCTTGG). The nucleotides may be in any order. The sequence may separate the generic sequence from the third portion of the primer.

Preferably at least a part of the first portion and second portion of the or each second primer is susceptible to degradation to which at least the third portion and at least a part of the second portion of the primer are not susceptible; and the method further comprises the step of:
c) degrading the susceptible part of the or each primer from the amplicon.

This removes a region of known sequence from the amplicon and prevents re-formation of a stem (where a stem-loop structure is present) in the second portion of the second primer remnant in the amplicon. Re-formation of this stem could otherwise hamper access to the intended primer binding site of a further primer or primer pair at the 3' end of this primer or primer pair.

The method may further comprise the step of:
d) amplifying the product of b) and/or the product of c) with a third primer or primer pair, the or each third primer comprising a 3' nucleic acid sequence substantially identical (and preferably identical) to at least a portion of the or each second primer.

Preferably the substantially identical nucleic acid sequence of the third primer is substantially identical to the undegraded non-susceptible part of the or each second primer, thereby generating an amplified product comprising a region of interest and sequences of the undegraded parts of the or each second primer. The substantially identical portion may be substantially identical to a generic sequence comprised within the second portion of the second primer.

This method addresses the difficulties inherent with prior art methods. In particular, the second primer or primer pair (referred to as a "bubble primer", due to the loop or bubble formed on hybridisation) can incorporate two regions of known but varying sequence (complementary to the target, and hence varying depending on the target), and a region of fixed sequence, which is not complementary to the target. The fixed sequence can be used to introduce sequencing adaptors or other sequences of utility into the amplified region without the need for ligation reactions. The fixed sequence may be an artificial sequence or a sequence derived from another organism that is not complementary to the template. In preferred embodiments, the fixed sequence may comprise a generic sequence; for example, a sequencing primer sequence.

Where a portion or a sequence is described as "not substantially identical" to a target or to another sequence, preferably that portion or sequence is dissimilar to the target or other sequence, such that under the conditions used in the amplification reaction that portion or sequence does not hybridise to a sequence complementary to the target. Likewise, where a sequence is "not complementary" to a target, it is sufficiently dissimilar such that it does not hybridise to the target under the conditions used in the amplification reaction.

The portion which is "susceptible to degradation" may also be referred to herein as a "degradable portion", while the portion which is not susceptible to said degradation may be referred to herein as a "resistant portion". The terms are used interchangeably.

The template may be a genomic polynucleotide. The template may be eukaryotic, prokaryotic, or archaeal. One or more templates may be provided. The template may represent a fragment of a genome; for example, a single chromosome, or a single genomic locus (for example, for rapid sequencing of allelic polymorphisms).

Where there is a second primer pair (ie, consisting of primers A and B), the first and third portions of primer A will be distinct from those of primer B, while the second portion may be distinct or may be identical, but is preferably distinct. In different second primer pairs (ie, primer A and B; and primer A' and B'), the second portions of corresponding primers (A and A'; B and B') will be identical, but may nonetheless be distinct within each pair. The first and third portions give target specificity, and allow for asymmetric integration of the primers.

Further, the use of first and third portions of the second primer (or primer pair) allows for the bubble portion to be generated such that the first and third portions are in close proximity, and the primer(s) retain a high degree of specificity for the target in order to reduce the chances of non-specific hybridisation and amplification.

Preferably the first and second portions of the template are separated by 0-20 nucleotides, preferably 1-10, more preferably 1-6, and most preferably 1, 2, 3, 4, 5, or 6 nucleotides.

The first portion of the second primer (or primer pair) may be up to 15, 20, 25, 30, 35, 50 nucleotides in length, preferably 20-35 nucleotides, more preferably 25 nucleotides.

The second portion of the second primer (or primer pair) may comprise a first degradable portion and a second resistant portion. The first degradable portion is preferably adjacent the first portion of the primer, and the second resistant portion is adjacent the third portion of the primer.

The second portion of the second primer (or primer pair) preferably comprises a self-complementary region, such that the loop formed upon hybridisation takes a stem-loop structure in which the self-complementary region forms the stem. The formation of the stem draws the first and third portions of the primer together, forcing the third portion into intimate contact with its complementary sequence, if present as the second portion of the template DNA. The loop may be minimal in length (typically, around four nucleotides are needed to form a loop), but preferably the second region further comprises a non-self-complementary region forming a larger loop. Where the second portion comprises a degradable and a resistant portion, the degradable portion preferably forms one half of the stem, with the resistant portion forming the other half of the stem plus the loop.

The third portion of the second primer (or primer pair) is preferably no more than 2, 4, 5, 6, 7, 8, 9, or 10 nucleotides in length. A preferred size is no more than 6, and preferably 4 to 6, most preferably 6, nucleotides. This length is believed to provide sufficient specificity (together with the first portion) to the primer, while reducing the total length of non-informative nucleotides which must be sequenced in a subsequent sequencing reaction.

Preferably the second portion, or the second and third portions together, of the second primer (or primer pair) is or are selected so as to include a tetrad of nucleotides comprising all four of the nucleotide bases (A, C, G, T). The order of the nucleotides is not important. This allows calibration of a sequencing reaction by providing a known sequence having all four nucleotides at the start of the region to be sequenced. The tetrad may separate the second and third portions. Where the second portion comprises a degradable and a resistant portion, then the tetrad may be present in the resistant portion or the resistant portion together with the third portion. The tetrad of nucleotides is preferably situated immediately adjacent to the 3' end of a sequencing primer sequence. More than four nucleotides may be included, provided each nucleotide is present in known numbers (for example, the sequence may be AAGGCCTT).

The degradable portions of the second primer pair may comprise RNA, while the resistant portions comprise DNA. Alternatively, the degradable portions may comprise DNA in which thymine has been replaced with uracil. The degradable portions may thus be degraded by RNAse H or alkaline pyrolysis (for RNA), or by uracil-N-glycosylase (for U-containing DNA), each of which normal DNA is resistant to.

The third primer pair in step d) may further comprise additional non-template sequences at the 5' end; this allows incorporation of additional functional sequences into the amplicon. For example, the additional sequences may include selectable markers, tags for purification or detection, moieties for physical capture of amplicons, clonal amplification sequences, or the like.

The first primer pair may be selected such that the 3' end of each primer is 5'-wards of the region corresponding to the third portion of each respective corresponding primer of the second primer or primer pair. That is, the second primers (also called 'bubble primers') are nested, and the amplification is nested PCR.

A further aspect of the disclosure provides a primer, the primer comprising a nucleic acid sequence having a first portion which is complementary to a first portion of a starting template for amplification, a second portion which is not complementary to the starting template, and a third portion which is complementary to a second portion of the starting template;
wherein the first and second portions of the starting template are adjacent or in close proximity to one another;
wherein the first, second, and third portions of the primer are arranged in that order from 5' to 3', such that on hybridisation to the starting template the second portion of the primer remains unhybridised and forms a loop between the first and third portions.

Here the starting template refers to the sequence which will be amplified by this primer, and which in the method defined above will have been initially amplified by a conventional primer pair to generate an amplicon.

Preferably also at least a part of the first portion and second portion of each primer is susceptible to degradation to which at least the third portion and at least a part of the second portion of the primer are not susceptible.

Also provided is a primer pair comprising a pair or primers as described above.

Although PCR is likely the most widely used amplification method and is used by way of example here, other non-thermocycling methods of amplification may be envisaged.

A still further aspect of the disclosure provides a library of primer pairs as herein described, the library comprising multiple primer pairs, each pair having first and second primers, comprising respective first and second second portions, wherein each first second portion is identical, and each second second portion is identical. The first and third portions may differ between primer pairs (and will be different in each primer of the primer pair).

### BRIEF DESCRIPTION OF THE DRAWINGS

Figure 1 shows a schematic illustration of a primer for use in the methods described herein.
Figure 2 illustrates the method for generating sequencing-ready polynucleotide fragments.

### DETAILED DESCRIPTION OF THE INVENTION

The methods disclosed herein enable the generation of NGS (next generation sequencing) "sequence-ready" DNA fragments that are a targeted subset of the total DNA present in the original template DNA sample. Just those loci of interest are amplified by, for example, polymerase chain reaction, such that the amplicons produced have the template DNA of interest flanked by terminal ends of known sequence. These known sequences are identical or substantially identical on all the amplicons generated, and are deliberately and controllably asymmetric, with two distinct sequences applied to each of the two ends of the amplified fragments. The amplicons thus produced are functionally equivalent to adapter-ligated fragments produced in conventional NGS methods, but offer distinct advantages in terms of ease, time and cost of production, as well as quality of the sequencing data subsequently produced. The terminal ends of the amplicons are amenable to generic 'one-size-fits-all' biochemistry during subsequent NGS manipulations, such as clonal amplification and DNA sequencing.

Further, embodiments of the methods enable a relatively short 3' end of a site-specific primer (the "third portion" in the summary of the invention) to hybridise in close proximity to a much larger, stably hybridised 5' element (the 'first portion' in the summary of invention) of the same primer, with these two target-complementary regions separated by a non-template sequence (the second portion in the summary of the invention) that will become part of the daughter amplicon upon successful primer extension. The non-template sequence will incorporate sequences of use in next generation sequencing, such that sequencing reactions can begin from that point. This minimises the amount of known DNA sequence data that would inevitably be wastefully generated from a direct 'adapter ligation' strategy, avoiding sequencing through a substantial 'region of no interest' amplification-primer remnant.

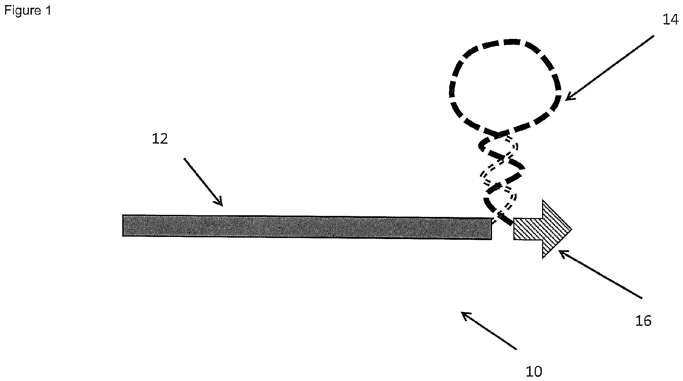

In addition, embodiments of the methods enable the use of NGS for the targeted analysis of specific genetic loci from within a complex DNA template source. Efficient targeted-panel sequencing is possible (for example, from a specific genetic locus or loci), rather than the current massively parallel 'whole genome shotgun sequencing'. An illustration of a primer for use in the method is shown in Figure 1. The primer 10 includes a first portion 12, a second portion 14, and a third portion 16. The first portion 12 is designed to be complementary to a part of the target genomic sequence to be amplified, while the third portion 16 is also designed to be complementary to an adjacent part of the target sequence. The first portion is around 25 nucleotides in length, with the third portion being around 6 nucleotides. There may be a gap of 0-4 nucleotides on the target between the sequences complementary to the first portion and those complementary to the third portion. This gap is to accommodate the non-complementary second portion 14 (the stem-loop structure) of the primer when first 12 and third 16 portions are hybridized to the target strand..

The second portion 14 is not complementary to the target, and includes a self-complementary region such that the sequence forms a stem-loop hairpin structure. The loop part and part of the stem of the second portion include sequences substantially identical to sequencing primers used in a chosen sequencing reaction. Note that the particular sequencing chemistry to be used is largely irrelevant; the method described herein is of general applicability, and is expected to be able to incorporate the relevant sequencing primer sequence into the amplicon. In certain embodiments the second portion may further comprise or be adjacent to a sequence comprising each of the four nucleotides A, C, G, T, in any order. Preferably the sequence is a tetrad (eg, ACGT), although the sequence may include multiple copies of each nucleotide, typically (but not necessarily) in equal numbers (eg, AAGGCCTT).

The primer 10 may include two types of nucleic acid. The first region, at the 5' end of the primer, may be sensitive to degradation by a selected technique, while the second region, at the 3' end of the primer, is insensitive to degradation by that technique. For example, the 5' end of the primer may be formed from RNA, while the 3' end is formed from DNA; the RNA portion may be degraded by RNAse H or alkaline pyrolysis, to which the DNA portion is resistant. Alternatively, the 5' end of the primer may be formed from DNA incorporating uracil in place of thymine; this will be degradable by uracil-N-glycosylase. In preferred embodiments, the degradable portion is degradable by an enzyme.

In this example, the degradable portion includes all of the first portion 12 of the primer, and a first section of the second portion 14 (shown on the second portion in double dashed line). The remainder of the primer is non-degradable. The degradable section of the second portion includes that region forming one half of the stem of the stem-loop structure; the non-degradable portion (shown in single dashed line) forms the loop and the second half of the stem adjacent the third portion. The non-degradable portion comprises a sequence that is at least substantially identical to the sequence of the sequencing primer. The sequencing primers hybridise to the complement of this sequence, produced upon DNA polymerisation (typically clonal amplification) generating the other strand.

The primer 10 may be used in a pair, consisting of forward and reverse primers. The forward and reverse primers include distinct first and third portions (as these are selected to be complementary to the endpoints of the region of the template to be amplified), and distinct second portions (leading to distinct forward and reverse sequencing primers being used), as the aim is to allow for asymmetric integration of the second portions into the amplicon. Where multiple primer pairs are provided, however, the second portions of each pair may be identical, to allow for common sequencing primers to be used to sequence all amplicons.

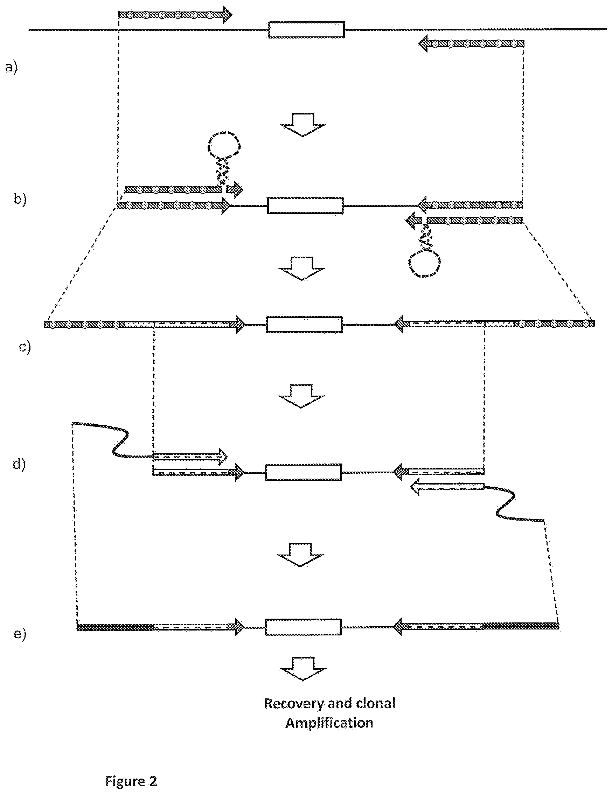

The method of generating amplicons using the primers is shown in Figure 2. This figure details the sequential steps performed in order to generate generic templates for a sequencing reaction in which a minimal amount of remnant primer sequences will be interrogated.

The method allows for the conversion of multiple separate template targets to products amenable to a generic sequencing workflow quickly, and with (ultimately) high sensitivity and specificity. The sequential amplification steps can be carried out discretely in separate amplification chambers, physically separating primer species, but one skilled in the art will appreciate that it may be possible to conduct these reactions in a smaller number of chambers (ideally just one) through selection of primer binding temperatures, careful control of primer concentrations (such that certain primer species are consumed to exhaustion) and by the application of a specific thermal cycling regime that temporally separates individual stages from participation in the overall process.

In the first step [Figure 2a], a standard PCR reaction is undertaken using conventional oligonucleotide primers, enriching the template population with the target of the amplification. This reaction can beneficially be carried out in multiplex, with distinct primer pairs delivering a relatively low specificity multiplex amplification of a number of different targets, to ensure that rare species are efficiently amplified. Low specificity primers in this initial phase may also to accommodate a degree of non-complementary base pairing within the targeted primer binding sites, as may be encountered in and around target DNA from cancer associated genes, for example. This initial amplification phase 2a can sacrifice specificity for enhanced sensitivity; tolerable as any inappropriately amplified species, including primer dimer artefacts, will be eliminated from further amplification during the subsequent stages. This step generates a first amplicon flanked by the primer sequences. Note that these primers may themselves be degradable (eg, formed from RNA, or from DNA incorporating U in place of T). These primers may be designed such that they will produce a limited amount of amplicon before becoming inefficient through one, or a combination, of;
- high Tm, with later cycles carried out at lower annealing temperature;
- low initial concentration of this primer

In step b), the amplicon from step a) is then amplified using the "bubble primers" or loop primers as described above. In Figure 2b, the novel Bubble Primer capitalises on the enriched pool of template generated during the first step 2a and efficiently propagates just those amplicons from 2a that were generated from the correct targets, rectifying that initial amplification may have been of relatively low specificity. This amplification therefore generates an amplicon pool that capitalises on the high sensitivity of the initial low specificity amplification (Figure 2a), but as the 3' end of the Bubble Primer will only be entertained by the correct amplicons, high specificity is reestablished at this second stage (Figure 2b). The only amplicons that contain the 'bubble sequence' of the Bubble Primer are generated from a reaction that is now (in combination) high sensitivity (2a) and high specificity (2b). Any other off target amplicons or artefacts that are generated will fail to be taken forward through the reaction scheme, as they will lack the necessary generic sequences defined within the non-template (artificial) bubble of the Bubble Primer.

The sequences of the bubble primers are selected such that the amplification is nested with respect to the amplification in step a); that is, the first portion of the bubble primers is substantially identical to the primers of step a), while the third portion is 3'-wards of the 3' end of the primers of step a). This means that the third portion contains sequences not represented in the primers of step a), and allows a selective 'nested' PCR of only those amplicons that were correctly generated during the initial amplification, which may therefore accommodate a degree of reduced specificity. The sequence of the second and/or third portion is also ideally selected such that it contains a tetrad including each of the four nucleotides (A, C, G, T). The tetrad of nucleotides is preferably situated immediately adjacent to the 3' end of the region at least substantially identical to the sequencing primer. The primers may also include "Index Codes" within the stem of the stem loop structure; for example, to identify and label products. As an example, an index code may be used to identify a specific product from a specific individual template. Alternatively, or in addition, the six bases of the third portion of the bubble primer, if sequenced, would normally be sufficient to identify the specific target that was being sequenced in a reasonable size multiplex.

Step c) shows the amplicon generated in step b). The amplification product has non-template sequences (that is, the sequences of the second portion of the bubble primer) represented in close proximity to the target DNA sequence. This product may have degradable sequence (eg, RNA) derived from the initial target-specific PCR binding sites, and the RNA-containing remnant of the non-template loop.

In step d), the product of step c) may be degraded (eg, by using RNAse H and/or RNAse A), to remove the degradable sequence if present from the amplicon. This degradation also removes any excess degradable primers which are not incorporated into the amplicon, functionally removing these from any further activity. The remaining amplicon therefore includes only the amplified target sequence incorporating the non-degradable, non-target sequence of the second portion and the third portion from the primers. Optionally at this stage, a generic PCR amplification may also be carried out with primers targeted to the non-target sequence of the bubble primers (referred to as a third primer pair in the "summary of invention" section above). These further primers may additionally carry a non-template artificial 5' extension for use as a sequence capture tag, a region used for clonal amplification, or for post-preamp amplification of the product.

Whether or not the 5' susceptible end of the amplicon generated is digested away, the next stage of the amplification scheme relies on the amplification of the target amplicons using a primer that is at least substantially identical to the non-template (artificial) sequence of the Bubble Primer. All amplicons that are generated within a multiplex reaction are amenable to amplification in a generic fashion using this primer, at least substantially identical to the artificial sequence provided within the non-template region of the Bubble Primer. This generic primer acts as an amplification primer, whereas a primer with identical or substantially identical sequence can be used as the ultimate 'sequencing primer' during the sequencing reaction, with the 3' end of the sequencing primer placed (generically) close to the region of the target amplicons to be interrogated, separated only by the few target-specific bases (ideally a number of between 4 and 10 bases, with 6 bases, 7 bases or 8 bases being most desirable, depending on GC content of this template-defined region). The region between the 3' end of the generic sequencing primer and the target-specific bases are designed or selected to include a tetrad of nucleotides A, T, G and C to act as a primer of the level of signal generated from each of these single base incorporation events. This nucleotide tetrad may be provided as polynucleotide representations of each of the nucleotide types (AA, TT, GG and CC or AAA, TTT, GGG, or CCC for example). The order of presentation of the bases within the tetrad primer is not important, and the number of representations of each base can be varied (e.g. AA, TTT, GG, CCC).

Step e) shows the final product. This includes the target sequence optionally flanked by a sequence available for capture/ clonal amplification (introduced in the amplification in step d)); a region available for hybridisation of a generic sequencing primer (derived from within the second portion of the bubble primer) and a region (derived from within the third portion, or between the second and third portions of the bubble primer) harbouring A, T, G and C to act as a reference for the signal strength generated for each base incorporation during sequencing. The final product may then be recovered, and used in a sequencing reaction.

The generic amplification of the target sequences using a primer at least substantially identical to the non-template sequence of the Bubble Primer can benefit from the inclusion of generic 5' tag tail extensions, which can be used to capture individual molecules of the multiplex amplicon pool and facilitate the clonal amplification of these individual molecules in (again) a generic fashion. One skilled in the art will recognise that the reliance on amplifications that are based on artificial sequences gives tremendous scope for the target-specific or general optimisation of these amplifications and that the overall scheme will produce a population of amplicons that are amenable to sequencing that is NGS technology agnostic.

The method described herein delivers a pool of 'end modified' fragments that have consistent (reliable asymmetric) adapter sequences attached to the ends, as opposed to the -50% randomly symmetrical products achieved by adapter ligation strategies: symmetrical products are not amenable to supporting clonal amplification for NGS sequencing and the invention therefore effectively eliminates the reduction in available template of utility in NGS.

The method enables the rapid generation of a pool of short fragments of DNA in which the interior of the fragments is the DNA sequence of interest, to be determined by NGS, and the ends of the fragments are substantially generic, allowing parallel processing during the generation of the clonal populations required for signal enhancement.

The method uses primer designs that, in one embodiment, employ the replacement of thymine bases with uracil bases, enabling functional removal of these sequences to the advantage of the efficient production of the desired products. In another embodiment, the use of primer designs that are a hybrid of RNA at the 5' end of the primer, and DNA at the 3' end of the primer, enabling digestion of the RNA component when hybridised to DNA, and the functional removal of this component.

The 3' end of the bubble primers, the third portion, includes a limited number of template-specific bases, sufficient to entertain DNA polymerase attachment and extension, but limiting the number of bases that will be 'wastefully' represented and sequenced in the final product used for NGS reactions.

The methods and primers described herein have a number of advantages over the prior art. In some embodiments, the attachment of sequences of DNA to the ends of specific regions of DNA enables these different regions to be analysed in multiplex, with the same applied biochemistry effecting NGS sequencing in parallel-processing. The methods and primers provide generic regions on the end of targeted DNA regions, the generic regions being available to support capture and clonal amplification of a diversity of targeted regions on a diversity of solid and/ or aqueous phases. Further, the methods and primers circumvent the need to use ligation of DNA adapters to the ends of fragments of DNA generated by DNA amplification, and provides template amenable for efficient sequencing.

The methods and primers are agnostic over the subsequent manipulations that generate pools of clonally amplified products (amenable to the generation of clonal populations both on a surface, on a bead or in solution). The technology is also agnostic of the technology that is subsequently used to generate the NGS data, and could be used (for example) with Illumina SBS technology, Ion Torrent or Roche 454 'one base at a time' technologies, or other NGS technologies such as nanopore sequencing. In general, the methods described herein may be advantageous where it is desirable to introduce defined sequences onto the end or ends of specific amplified products.

The methods and primers are of principal utility in the analysis of a panel of DNA targets selected from a much larger available pool of DNA sequences.

## Claims

1. A method for generating and sequencing polynucleotide fragments from a starting template polynucleotide, the method comprising:
a) amplifying a region of interest from the starting template using a first primer pair to form an amplicon incorporating the region of interest,
b) amplifying the region of interest from the first amplicon generated in step a) using a nucleic acid amplification reaction with a second primer, to form an amplicon incorporating the second primer,
wherein the second primer comprises a nucleic acid sequence having a first portion which is complementary to a first portion of the starting template, a second portion which is not complementary to the starting template, and a third portion which is complementary to a second portion of the starting template;
wherein the first and second portions of the starting template are adjacent or in close proximity to one another;
wherein the first, second, and third portions of the second primer are arranged in that order from 5' to 3', such that on hybridisation to the starting template the second portion of the primer remains unhybridised and forms a loop between the first and third portions;
thereby generating an amplified product comprising a region of interest flanked by sequences of the second primer, wherein the second portion of the second primer comprises a generic sequence, and
c) sequencing the generated amplified product using a sequencing primer hybridised to the complement of the generic sequence, of the second portion of the second primer.

2. The method of claim 1, wherein at least a part of the first portion of the or each second primer is susceptible to degradation to which at least the third portion and at least a part of the second portion of the primer are not susceptible; and the method further comprises the step of:
d) degrading the susceptible part of the or each primer from the amplicon.

3. The method of claim 1 or claim 2, further comprising the step of:
e) amplifying the product of b) and/or the product of d) with a third primer pair, each primer comprising a nucleic acid sequence substantially identical to at least a portion of the second portion of the or each second primer.

4. The method of claim 3, wherein the product of b) and/or the product of d) is amplified, and at least a portion of the nucleic acid sequence of the third primer is substantially identical to the generic sequence of the second portion of the or each second primer.

5. The method of any preceding claim, wherein the first and second portions of the template are separated by 0-20 nucleotides, preferably 1-10, more preferably 1-6, and most preferably 1, 2, 3, 4, 5, or 6 nucleotides.

6. The method of any preceding claim, wherein the first portion of the second primer is up to 15, 20, 25, 30, 35, 50 nucleotides in length, preferably 20-35 nucleotides, more preferably 25 nucleotides.

7. The method of any preceding claim, wherein the second portion of the second primer comprises a self-complementary region, such that the loop formed upon hybridisation takes a stem-loop structure in which the self-complementary region forms the stem.

8. The method of any preceding claim, wherein the third portion of the second primer is no more than 2, 4, 5, 6, 7, 8, 9, or 10 nucleotides in length, preferably 4 to 6, most preferably 6.

9. The method of claim 3, wherein the third primer pair in step d) further comprises additional non-template sequences at the 5' end.

## Patentansprüche

1. Verfahren zum Erzeugen und Sequenzieren von Polynukleotidfragmenten aus einem Rohmatrizenpolynukleotid, wobei das Verfahren Folgendes umfasst:
a) Verstärken einer Region von Interesse von der Rohmatrize unter Verwendung eines ersten Primer-Paares, um ein Amplikon zu bilden, das die Region von Interesse enthält,
b) Verstärken der Region von Interesse von dem ersten Amplikon, das in Schritt a) erzeugt wird, unter Anwendung einer Nukleinsäureverstärkungsreaktion mit einem zweiten Primer, um ein Amplikon zu bilden, das den zweiten Primer enthält,
wobei der zweite Primer eine Nukleinsäuresequenz umfasst, die einen ersten Abschnitt, der komplementär zu einem ersten Abschnitt der Rohmatrize ist, einen zweiten Abschnitt, der nicht komplementär zu der Rohmatrize ist, und einen dritten Abschnitt aufweist, der komplementär zu einem zweiten Abschnitt der Rohmatrize ist;
wobei der erste und zweite Abschnitt der Rohmatrize benachbart oder in enger Nähe zueinander sind;
wobei der erste, zweite und dritte Abschnitt des zweiten Primers in der Reihenfolge von 5' zu 3' angeordnet sind, sodass der zweite Abschnitt des Primers bei Hybridisierung zu der Rohmatrize unhybridisiert bleibt und eine Schleife zwischen dem ersten und dritten Abschnitt bildet;
wodurch ein verstärktes Produkt erzeugt wird, das eine Region von Interesse flankiert durch Sequenzen des zweiten Primers umfasst, wobei der zweite Abschnitt des zweiten Primers eine generische Sequenz umfasst, und
c) Sequenzieren des erzeugten verstärkten Produktes unter Verwendung eines Sequenzierungsprimers, der zu dem Komplement der generischen Sequenz hybridisiert ist, des zweiten Abschnitts des zweiten Primers.

2. Verfahren nach Anspruch 1, wobei zumindest ein Teil des ersten Abschnitts des oder jedes zweiten Primers anfällig für Degradation ist, wofür zumindest der dritte Abschnitt und zumindest ein Teil des zweiten Abschnitts des Primers nicht anfällig sind; und das Verfahren ferner den folgenden Schritt umfasst:
d) Degradieren des anfälligen Teils des oder jedes Primers von dem Amplikon.

3. Verfahren nach Anspruch 1 oder Anspruch 2, ferner den folgenden Schritt umfassend:
e) Verstärken des Produkts aus b) und/oder des Produkts aus d) mit einem dritten Primer-Paar, wobei jeder Primer eine Nukleinsäuresequenz umfasst, die mit zumindest einem Abschnitt des zweiten Abschnitts des oder jedes zweiten Primers im Wesentlichen identisch ist.

4. Verfahren nach Anspruch 3, wobei das Produkt aus b) und/oder das Produkt aus d) verstärkt wird und zumindest ein Abschnitt der Nukleinsäuresequenz des dritten Primers mit der generischen Sequenz des zweiten Abschnitts des oder jedes zweiten Primers im Wesentlichen identisch ist.

5. Verfahren nach einem vorhergehenden Anspruch, wobei der erste und zweite Abschnitt der Matrize durch 0-20 Nukleotide, bevorzugt 1-10, bevorzugter 1-6 und am meisten bevorzugt 1, 2, 3, 4, 5 oder 6 Nukleotide getrennt sind.

6. Verfahren nach einem vorhergehenden Anspruch, wobei der erste Abschnitt des zweiten Primers eine Länge von bis zu 15, 20, 25, 30, 35, 50 Nukleotiden, bevorzugt 20-35 Nukleotiden, bevorzugter 25 Nukleotiden aufweist.

7. Verfahren nach einem vorhergehenden Anspruch, wobei der zweite Abschnitt des zweiten Primers eine selbstkomplementäre Region umfasst, sodass die bei Hybridisierung gebildete Schleife eine Stamm-Schleifen-Struktur annimmt, in der die selbstkomplementäre Region den Stamm bildet.

8. Verfahren nach einem vorhergehenden Anspruch, wobei der dritte Abschnitt des zweiten Primers eine Länge von nicht mehr als 2, 4, 5, 6, 7, 8, 9 oder 10 Nukleotiden, bevorzugt 4 bis 6, am meisten bevorzugt 6 aufweist.

9. Verfahren nach Anspruch 3, wobei das dritte Primer-Paar in Schritt d) ferner zusätzliche Nichtmatrizensequenzen an dem 5'-Ende umfasst.

## Revendications

1. Procédé de génération et de séquençage de fragments de polynucléotide à partir d'un polynucléotide de modèle de départ, le procédé comprenant :
a) l'amplification d'une région d'intérêt à partir du modèle de départ en utilisant une première paire d'amorces afin de former un amplicon incorporant la région d'intérêt,
b) l'amplification de la région d'intérêt à partir du premier amplicon généré dans l'étape a) en utilisant une réaction d'amplification d'acide nucléique avec une deuxième amorce, afin de former un amplicon incorporant la deuxième amorce, dans lequel la deuxième amorce comprend une séquence d'acides nucléiques comportant une première partie qui est complémentaire à une première partie du modèle de départ, une deuxième partie qui n'est pas complémentaire au modèle de départ, et une troisième partie qui est complémentaire à une deuxième partie du modèle de départ ;
dans lequel les première et deuxième parties du modèle de départ sont adjacentes ou à proximité immédiate l'une de l'autre ;
dans lequel les première, deuxième et troisième parties de la deuxième amorce sont agencées dans cet ordre de l'extrémité 5' à l'extrémité 3', de sorte qu'au moment de l'hybridation sur le modèle de départ la deuxième partie de l'amorce reste non hybridée et forme une boucle entre les première et troisième parties ;
permettant ainsi de générer un produit amplifié comprenant une région d'intérêt flanquée par les séquences de la deuxième amorce, dans lequel la deuxième partie de la deuxième amorce comprend une séquence générique, et
c) le séquençage du produit amplifié généré en utilisant une amorce de séquençage hybridée au complément de la séquence générique, de la deuxième partie de la deuxième amorce.

2. Procédé selon la revendication 1, dans lequel au moins une partie de la première partie de la deuxième amorce ou de chaque deuxième amorce est sujette à la dégradation à laquelle au moins la troisième partie et au moins une partie de la deuxième partie de l'amorce ne sont pas sujettes ; et le procédé comprend en outre l'étape de :
d) dégradation de la partie susceptible de l'amorce ou de chaque amorce de l'amplicon.

3. Procédé selon la revendication 1 ou la revendication 2, comprenant en outre l'étape de :
e) amplification du produit de b) et/ou du produit de d) avec une troisième paire d'amorces, chaque amorce comprenant une séquence d'acides nucléiques sensiblement identique à au moins une partie de la deuxième partie de la deuxième amorce ou de chaque deuxième amorce.

4. Procédé selon la revendication 3, dans lequel le produit de b) et/ou le produit de d) est amplifié, et au moins une partie de la séquence d'acides nucléiques de la troisième amorce est sensiblement identique à la séquence générique de la deuxième partie de la deuxième amorce ou de chaque deuxième amorce.

5. Procédé selon l'une quelconque des revendications précédentes, dans lequel les première et deuxième parties du modèle sont séparées par 0 à 20 nucléotides, de préférence par 1 à 10, plus préférablement par 1 à 6, et encore plus préférablement par 1, 2, 3, 4, 5, ou 6 nucléotides.

6. Procédé selon l'une quelconque des revendications précédentes, dans lequel la première partie de la deuxième amorce est constituée jusqu'à 15, 20, 25, 30, 35, 50 nucléotides en longueur, de préférence de 20 à 35 nucléotides, plus préférablement de 25 nucléotides.

7. Procédé selon l'une quelconque des revendications précédentes, dans lequel la deuxième partie de la deuxième amorce comprend une région auto-complémentaire, de sorte que la boucle formée après hybridation prend une structure de tige-boucle dans laquelle la région auto-complémentaire forme la tige.

8. Procédé selon l'une quelconque des revendications précédentes, dans lequel la troisième partie de la deuxième amorce comporte une longueur inférieure à 2, 4, 5, 6, 7, 8, 9, ou 10 nucléotides, de préférence de 4 à 6, le plus préférablement de 6.

9. Procédé selon la revendication 3, dans lequel la troisième paire d'amorces dans l'étape d) comprend en outre des séquences supplémentaires sans modèle au niveau de l'extrémité 5'.
